# EUROPEAN PATENT SPECIFICATION

(11) **EP 1 991 111 B1**
(45) Date of publication and mention of the grant of the patent: **06.04.2011**
(21) Application number: 07751306.7
(22) Date of filing: 20.02.2007
(51) Int. Cl.: A61B 5/00, A61L 27/52, C12Q 1/00

(54) **HYDROGEL FOR AN INTRAVENOUS AMPEROMETRIC BIOSENSOR**
WASSERGEL FÜR INTRAVENÖSEN AMPEROMETRISCHEN BIOSENSOR
HYDROGEL POUR UN BIOCAPTEUR AMPÉROMÉTRIQUE INTRAVEINEUX

(30) Priority: 27.02.2006 US 777254 P
(43) Date of publication of application: 19.11.2008
(73) Proprietor: Edwards Lifesciences Corporation, Irvine, CA 92614 (US)
(72) Inventor: ROCHE, Joelle, Irvine, CA 92612 (US); CURRY, Kenneth, Oceanside, CA 92056 (US)
(74) Representative: Müller-Boré & Partner Patentanwälte
(86) International application number: PCT/US2007/004536
(87) International publication number: WO 2007/100588

(56) References cited:
- US-A- 4 983 524
- MIAO Y ET AL: "Amperometric glucose biosensor based on immobilization of glucose oxidase in chitosan matrix cross-linked with glutaraldehyde" ELECTROANALYSIS, vol. 13, no. 4, March 2001 (2001-03), pages 347-349, XP002444045 ISSN: 1040-0397
- KRAJEWSKA BARBARA: "Application of chitin- and chitosan-based materials for enzyme immobilizations: a review" ENZYME AND MICROBIAL TECHNOLOGY, vol. 35, no. 2-3, 5 August 2004 (2004-08-05), pages 126-139, XP002444046 ISSN: 0141-0229

## Description

### Field of the Invention

The invention relates to amperometric biosensors for measuring blood chemistry. In particular, the invention relates to an intravenous amperometric biosensor.

### BACKGROUND

Amperometric biosensors are known in the medical industry for analyzing blood chemistry. Early biosensors, also known as enzyme electrodes, were first proposed by Clark and Lyons and implemented by Updike and Hicks. Enzyme electrodes typically include an oxidase enzyme, such as glucose oxidase, that is immobilized behind a dialysis membrane at the surface of an electrode. In the presence of blood, the membrane selectively passes an analyte of interest, e.g. glucose, to the oxidase enzyme where it undergoes oxidation or reduction, e.g. the oxidation of glucose to gluconolactone. Amperometric biosensors function by producing an electric current when a potential sufficient to sustain the reaction is applied between two electrodes in the presence of the reactants. For example, in the reaction of glucose and glucose oxidase, the hydrogen peroxide byproduct may be subsequently oxidized by electron transfer to an electrode. The resulting flow of electrical current in the electrode is indicative of the concentration of the analyte of interest.

Applications for amperometric biosensors include measuring analytes in body fluids, electrolyte levels in blood and in particular, blood glucose concentration. For measuring glucose, subcutaneous methods have been proposed. For example, see Garg et al., "Improvement in Glycemic Excursions With a Transcutaneous, Real-Time Continuous Glucose Sensor," Diabetes Care, January 2006. While these minimally invasive glucose monitoring systems properly display trends in plasma glucose concentration, they do not track glucose accurately enough to be used for intensive insulin therapy for example, where inaccuracy at conditions of hypoglycemia could pose a very high risk to the patient. In addition, sensors based upon the enzyme glucose oxidase must have access to adequate oxygen to provide a linear glucose response. Sensor systems optimized for subcutaneous tissue would not necessarily function well in venous blood, where oxygen tension can be 20 mm Hg or less.

At the present time, the most reliable way to obtain a highly accurate blood glucose measurement in an ICU patient is by a direct time-point method, which involves drawing a blood sample and sending it off for laboratory analysis. This is a time-consuming method that is often incapable of producing needed results in a timely manner. Despite ongoing research in this field, many improvements in glucose monitoring are still needed.

One of the difficulties impeding the development of an intravenous amperometric sensor is that the sensor must be small enough to be suspended within a blood vessel, but robust enough to immobilize an enzyme so that a reaction may be sustained for a sufficient length of time. An intravenous sensor must also be biocompatible, such that it does not release any toxins into a patient, and when implanted, e.g. through a catheter in a femoral vein, discourages clotting of blood at the membrane surface that would prevent plasma from diffusing to the enzyme layer.

Miao et al describes a glucose biosensor fabricated using a cross linked chitosan film, where glutanaldehyde was used as cross-linker.

### SUMMARY

The invention is defined by the appended claims discloses novel hydrogel for use with an amperometric biosensor designed for intravenous use and continuous analyte monitoring. On a sensor having an electrode, the hydrogel may be formed as a layer having at least one reactive enzyme. The hydrogel layer may include an immobilizing matrix of chitosan cross-linked with at least one additional cross-linking agent to immobilize the reactive enzyme throughout the layer. In one embodiment, the reactive enzyme may include an oxidase such as glucose oxidase. In another embodiment, genipin is used as a cross-linking agent. In other embodiments, the sensor may be a thin-film sensor and the electrode may be a platinum electrode. The hydrogel itself may function as a rate-limiting layer, to selectively control a rate of diffusion to the hydrogel layer.

A related method is also disclosed for coating an electrode with a hydrogel layer having an immobile oxidase for use in an amperometric biosensor. The method may include dissolving chitosan in an acidic solution, adding an oxidase to the chitosan solution, applying the chitosan solution to a surface of the electrode, curing the chitosan solution until it forms a solid film, and immersing the solid film in a genipin solution. In one embodiment, the method may include cross-linking the chitosan with genipin to effectively immobilize the oxidase.

### BRIEF DESCRIPTION OF THE DRAWINGS

The features, objects, and advantages of the invention will become more apparent from the detailed description set forth below when taken in conjunction with the drawings, wherein:

FIG. 1 is an amperometric biosensor in the form of a flex circuit having a working electrode coated with a thin-film hydrogel layer according to an embodiment of the invention.

FIG. 2 is a magnified side view of the working electrode portion of the biosensor of FIG. 1, showing a hydrogel layer on a surface of the working electrode according to an embodiment of the invention.

FIG. 3 is a magnified side view of the working electrode portion of the biosensor of FIG. 1, showing a diffusion layer on a surface of the hydrogel layer according to an embodiment of the invention.

FIG. 4 is a process flow chart illustrating a method for manufacturing a hydrogel layer according to an embodiment of the invention.

FIG. 5 is a graph of glucose assay results of current output vs. glucose concentration for biosensors formed with hydrogel layers according to an embodiment of the invention.

FIG. 6 is a graph of glucose assay results of current output over time covering multiple step changes in glucose concentration, for biosensors formed with hydrogel layers according to an embodiment of the invention.

### DETAILED DESCRIPTION

The invention discloses an enzyme-immobilizing, robust, biocompatible hydrogel ideal for use as a reactive enzyme layer on a working electrode in an intravenous amperometric biosensor. The hydrogel may be formed from (i) chitosan, (ii) a reactive enzyme, and (iii) a cross-linking agent. As disclosed herein, this combination, has been proven to provide a mechanically strong cross-linked matrix that immobilizes the reactive enzyme and evenly distributes it throughout the hydrogel. In one embodiment, chitosan may be cross-linked with genipin to immobilize an oxidase such as glucose oxidase. Cross-linking in the hydrogel strengthens the formation and prevents it from dissolving in an aqueous solution.

One application for a hydrogel is in a thin-film amperometric biosensor formed on a flex circuit. Flex circuits have been used in the micro-electronics industry for many years. More recently, flex circuits have been applied in medical devices as microelectrode substrates for in vivo applications. For example, one flex circuit design uses a laminate of a conductive foil (e.g., copper) on a flexible dielectric substrate (e.g., polyimide). The flex circuit may be formed on the conductive foil using masking and photolithography techniques. Flex circuits are desirable due to their low manufacturing cost, ease in design integration, and physical flexibility during transport in applications such as central venous catheter (CVC) insertion.

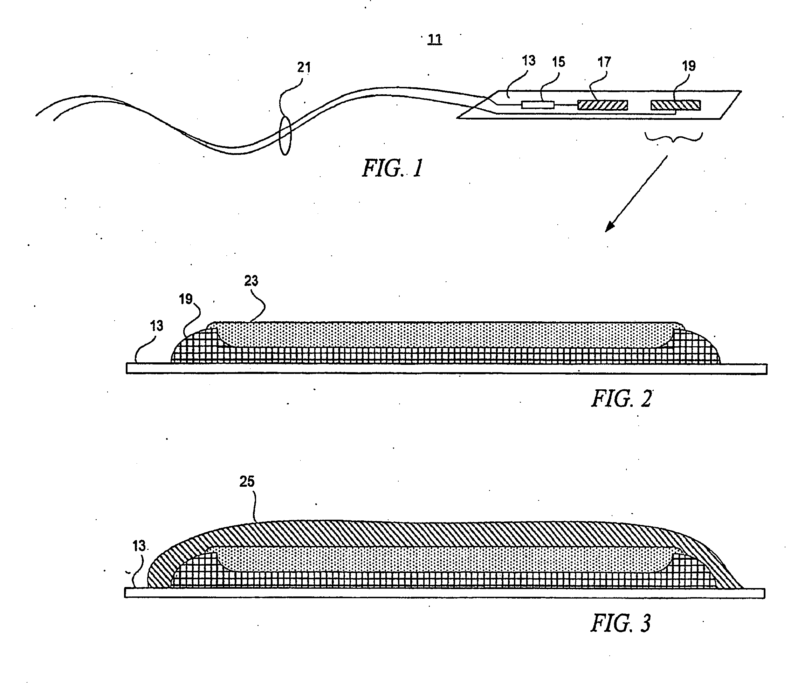

FIG. 1 is an amperometric biosensor 11 in the form of a flex circuit that incorporates a hydrogel layer according to an embodiment of the invention. The biosensor or sensor 11 may be formed on a substrate 13 (e.g., a flex substrate). One or more electrodes 15, 17 and 19 may be attached or bonded to a surface of the substrate 13. The biosensor 11 is shown with a reference electrode 15, a counter electrode 17, and a working electrode 19. In another embodiment, one or more additional working electrodes may be included on the substrate 13. Electrical wires 21 may transmit power to the electrodes for sustaining an oxidation or reduction reaction, and may also carry signal currents to a detection circuit (not shown) indicative of a parameter being measured. The parameter being measured may be any analyte of interest that occurs in, or may be derived from, blood chemistry. In one embodiment, the analyte of interest may be hydrogen peroxide, formed from reaction of glucose with glucose oxidase, thus having a concentration that is proportional to blood glucose concentration.

In one embodiment, the hydrogel layer may contain a glucose oxidase enzyme for use on a glucose sensor. The sensor 11 may include reference, counter, and working electrodes 15, 17 and 19, respectively. The working electrode 19 may be at least partially coated with a layer of the hydrogel, and is exposed to or immersed within a solution, such as blood, to allow the enzyme to chemically react with certain reactants in the blood. In this example, the glucose oxidase enzyme on the working electrode 19 reacts with glucose in the blood.

The sensor 11 works on an amperometric measurement principle, where the working electrode 19 is held at a positive potential relative to the counter electrode 17. The positive potential is sufficient to sustain an oxidation reaction of hydrogen peroxide, which is the result of glucose reaction with glucose oxidase. Thus, the working electrode 19 functions as an anode, and collects electrons produced at the surface of the working electrode 19 that result from the oxidation reaction. The collected electrons flow into the working electrode 19 as an electrical current. In one embodiment, with the working electrode coated with glucose oxidase, the oxidation of glucose produces a hydrogen peroxide molecule for every molecule of glucose when the working electrode 19 is held at a potential between about +450 mV and about +650 mV. The hydrogen peroxide produced oxidizes at the surface of the working electrode 19 according to the equation:

H₂O₂⁻¹→ 2H⁺ + O₂⁰ + 2e⁻

The equation indicates that two electrons are produced for every hydrogen peroxide molecule oxidized. Thus, under certain conditions, the amount of electrical current may be proportional to the hydrogen peroxide concentration. Since one hydrogen peroxide molecule is produced for every glucose molecule oxidized at the working electrode, a linear relationship may exist between the blood glucose concentration and the resulting electrical current. The reader may refer to the following article for additional information on electronic sensing theory for amperometric glucose biosensors: J. Wang, "Glucose Biosensors: 40 Years of Advances and Challenges," Electroanaylsis, Vol. 13, No. 12, pp. 983-988 (2001).

To ensure that conditions are favorable to achieve the linear relationship, the working electrode 19 is designed to promote the desired chemical reactions. In the amperometric sensor 11, the chemistry may be controlled by applying one or more membranes, or layers, of varying composition on the surface of a flex circuit substrate. In one embodiment, the substrate 13 may be a polyimide material and a membrane may be a hydrogel layer. The substrate 13 provides an insulated structure for mounting the electrodes and membrane layers. In one embodiment, on a flex circuit intended for use in a confined space, such as within a catheter lumen, the substrate 13 may have a width between about 0.015 inches and about 0.080 inches, and a length of about 1.0 to 2.0 inches. The thickness of the membrane layers may vary between about 0.5 microns and about 10 microns. In one embodiment, one or more of the membrane layers may have a thickness in the about 4 micron to about 5 micron range.

The electrical wires 21 may be coupled or soldered to conductive traces formed on the substrate 13 using conventional flex circuit technology. For example, the traces may be gold-plated copper. In one embodiment, the sensor 11 may be designed so that the flex circuit terminates to a tab that mates to a conventional multi-pin connector, such as a 3-pin, 1 mm pitch ZIF Molex connector. Such a connection facilitates excitation of the working electrode and measurement of electrical current signals, for example, using a potentiostat or other controller.

The electrodes 15, 17 and 19 may be applied to the substrate 13 using a thick film process and commercially available inks. In one embodiment, the reference electrode 15 may be a silver/silver chloride type deposited or formed on the substrate 13. The reference electrode 15 establishes a fixed potential from which the potential of the counter electrode 17 and the working electrode 19 may be established. The reference potential is Nernstian. For the silver/silver chloride electrode, the reference potential is maintained by the following half-reaction:

Ag⁰ → Ag⁺ + e⁻

The counter electrode 17 may be constructed from conductive materials such as platinum or graphite. These materials may be formulated as an ink for application to the substrate 13 using a thick film process and cured accordingly. The counter electrode 17 provides a working area for conducting the majority of electrons produced from the oxidation chemistry back to the blood solution. Otherwise, all the current would likely pass through the reference electrode 15, and may reduce its service life. In one embodiment, the counter electrode 17 may be formed with a surface area greater than that of the working electrode 19.

The working electrode 19 may be formed using platinum/graphite materials similar to those used for forming the counter electrode 17. In other embodiments, the working electrode 19 may be formed from other conductive materials. Its operation has been described thus far as promoting anodic oxidation of hydrogen peroxide at its surface. Other embodiments are possible, however, the working electrode 19 may be held at a negative potential. In this case, the electrical current produced at the working electrode 19 may result from reduction of oxygen.

In one embodiment, the biosensor 11 may be installed within a probe or catheter for intravenous insertion into a patient, for example, via a CVC. The biosensor 11 may function as an amperometric sensor while immersed in a patient's bloodstream by the addition of a reactive hydrogel layer 23 to a surface of the working electrode 19. The hydrogel layer 23 is made reactive by the addition of a reactive agent to the base material of the layer.

The magnified side view of FIG. 2 shows an illustration of a hydrogel layer 23 formed on the working electrode 19. The figure shows a distal portion of the substrate 13 in the vicinity of the working electrode 19. In another embodiment as shown in FIG. 3, a rate-limiting layer 25 may be added on top of the hydrogel layer 23 to selectively allow diffusion to the hydrogel layer 23 of a blood component that reacts with the reactive agent.

The hydrogel layer 23 may be composed of chitosan, at least one reactive agent, and at least one cross-linking agent. The at least one cross-linking agent cross-links to the chitosan to form a matrix that immobilizes the at least one reactive agent. The chitosan, the reactive agent, and the cross-linking agent may be substantially uniformly distributed throughout the resulting hydrogel.

The chitosan is derived from chitin, the structural element in the exoskeletons of crustaceans, and is one of the most common polysaccharides on earth. The chitosan is chosen as a base material for the hydrogel layer 23 based on its biocompatibility, its availability, its cross-linking affinity, and for its adhesion properties. The chitosan is non-toxic, and has been found to discourage platelet adhesion or thrombus on the surface of an implanted sensor. In addition, the chitosan may be made to form a hard, clear layer on a metal surface, and has been used effectively as a protective spray coating on metal bridges in saltwater environments.

The hydrogel layer 23 includes at least one reactive agent that may be added to the chitosan. In one embodiment, the reactive agent may be an enzyme such as an oxidase enzyme. In an embodiment for a glucose biosensor, the reactive agent may be glucose oxidase, such as may be derived from Aspergillus niger (EC 1.1.3.4), type II or type VII. To promote a reaction of the reactive agent with blood, the reactive agent may be distributed evenly throughout the hydrogel layer, so that some amount of the reactive agent is exposed to the hydrogel surface. This may be achieved by adding or cross-linking the reactive agent to the chitosan. The hydrogel layer 23 may be water absorbent, so that it may swell to provide active transport of a reactant in the blood (e.g. glucose) from the blood to the reactive agent. Thus, intermolecular bonds may be formed throughout the hydrogel layer 23 to create adhesion and a density of matrix to allow for even dispersion of the reactive agent across the hydrogel surface and throughout the hydrogel layer 23. Reaction products may then be communicated to the electrode layer.

Chitosan by itself, however, may be insufficient to provide an adequate robustness needed to contain the reactive agent. Therefore, a cross-linking agent such as genipin may be added to the chitosan to form a matrix with sufficient mechanical strength and immobilizing properties. Genipin, the active compound found in gardenia fruit extract, is an herbal remedy used in traditional Chinese medicine. Genipin is a biocompatible and non-toxic cross-linking agent that when in the body, breaks down harmlessly into carbon dioxide and water.

Cross-linking genipin to chitosan ensures mechanical robustness of hydrogel layer 23, so that it remains intact on the surface of the working electrode 19. This property is important to ensure the integrity of the hydrogel layer 23 during manufacturing, and also during installation in vivo, for example, during transport to a measuring location via a CVC catheter. The cross-linking compatibility of genipin with chitosan has been documented in J. Berger, et al., "Structure and Interactions in Covalently and Ionically Crosslinked Chitosan Hydrogels for Biomedical Applications," Europ. J. Pharm. Biopharm., Vol. 57, No. 1, pp. 19-34 (2004).

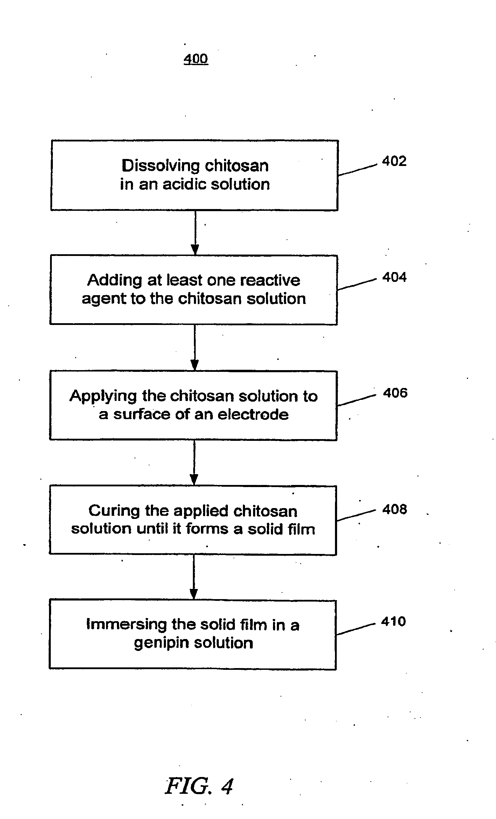

Based on experimental trials, the substance of which is disclosed herein, a proven method has been developed and is herein disclosed as a method or process of forming a hydrogel for immobilizing a reactive agent on an electrode. FIG. 4 illustrates one such embodiment of a method 400.

In step 402, chitosan is dissolved in an acetic acid solution. For example, in one embodiment, the chitosan may be dissolved in a solution of acetic acid to create a chitosan concentration of between about 0.1 and about 2.0 weight percent. Alternatively, chitosan may be dissolved in an acetic acid solution having a concentration of between about 1 percent and 5 percent. Step 402 may include stirring or agitating the solution until the chitosan is dissolved. In step 404, at least one reactive agent such as an oxidase or other enzyme may be added to the chitosan solution. Step 404 may also include agitating or mixing the solution, and may proceed until the solution forms a viscous liquid or gel. In one embodiment, step 404 may yield an oxidase concentration of between about 1/120,000 and about 1/10,000 weight percent oxidase (with respect to the solution).

In step 406, the chitosan solution or gel may be applied to a surface of an electrode. The application of the chitosan may partially or completely coat the surface with a substantially uniform layer. In step 408, the applied chitosan may be cured until it forms a solid film on the electrode surface. Step 408 may be performed at room temperature or under low heat. In step 410, the reactive agent is immobilized in the hydrogel layer. This may be accomplished by immersing the solid film in a genipin solution to promote cross-linking of genipin to the chitosan. In one embodiment, the film-coated electrode may be immersed for between about 2 and about 10 hours. In other embodiments, the genipin solution may be a phosphate or citrate buffer, having about one percent weight-to-volume of genipin, and having a pH value of between about 4 and about 10. Optionally, after curing the cross-linked genipin and chitosan, a second, more densely cross-linked layer of genipin and chitosan may be added to the cured cross-linked genipin and chitosan to provide a rate-limiting layer for selectively diffusing a blood component to react with the reactive agent.

EXPERIMENTAL RESULTS

A population of prototype glucose sensors was fabricated in a laboratory using method 400 to immobilize glucose oxidase in a chitosan-genipin hydrogel formed on platinum electrodes. The glucose sensors were tested for various mechanical and chemical properties. The primary mechanical properties tested were adhesion, and adhesion after soaking. The primary chemical property tested was a glucose response. Type 7 glucose oxidase was used for the experiments. The following disclosure presents the test sensor fabrication procedures and the results obtained from the primary tests conducted on the test sensor population.

For all tests, test sensors were fabricated using a platinum ink, which was made and deposited on a sensor substrate in a 10 mm x 10mm patch. The platinum was applied using a carbon/graphite ink to ensure good adhesion to the flex substrate. After curing, the ink formed a solid platinum pad. The sensor substrate was a flex-sensor, epoxy layer deposited over a thin metallic layer, with a circuit trace etched into the flex, and wires joined thereto.

Adhesion tests were performed on a first population of test sensors to which a hydrogel was applied from a chitosan-glucose oxidase solution without genipin. Different formulations of 2000 µL solutions were made, all containing 0.25 wt. % chitosan in 1% acetic acid. Each solution was made by dissolving the chitosan in the acetic acid and stirring in an ice bath to form a viscous gel. The glucose oxidase was added and dissolved in the solution, forming a yellow gel material *- i.e.* the hydrogel. The amount of glucose oxidase was varied for different test sensors, and included hydrogel solutions made from 0, 1/160,000, 1/140,000, and 1/120,000 wt. % glucose oxidase. The hydrogel was applied to the dried platinum ink area and allowed to cure. Some test sensors were cured in ambient air, and others were cured in a low-temperature oven. After curing, the hydrogel layer formed a hard, clear film.

For the adhesion test, each 10 mm x 10 mm patch was scored to form 100 cubes, each cube 1 mm x 1 mm. An ASTM tape test standard was used to determine adhesion of the hydrogel to the platinum electrode surface. The tape was applied and removed, and the patch was inspected for the presence or absence of hydrogel cubes. The best results were obtained from air-dried hydrogel formed from 1/40,000 wt. % and 1/60,000 wt. % glucose oxidase.

The air-dried test sensors were then soaked in a phosphate buffer at body temperature with a pH of about 7.4. The purpose of the soak test was to test the durability of the hydrogel adhesion in a simulated intravenous environment. After immersion for a period of time, the adhesion test with tape was performed again. However, shortly after immersion, a dye cloud appeared in the buffer solution and all hydrogel layers appeared to dissolve.

A second adhesion test was performed using a second population of test sensors. In this test, the hydrogel was composed of chitosan, glucose oxidase, and genipin. The second population test sensors were fabricated nearly identically as the first population. The chitosan solution used for the second population of test sensors was 100 ml of 1.0 wt. % chitosan in 1% acetic acid. A hydrogel was prepared using 15 mg of glucose oxidase in 4 mL of the chitosan solution. Additional steps were then taken to add genipin to the hydrogel layer.

To cross-link the chitosan film to genipin, different pH levels of a genipin solution were tested. It was found that depending on the pH of the genipin, the genipin may cross-link to itself first (pH 7-10), forming a long chain that creates large bonds between genipin and chitosan, or at a lower pH (4-5) the genipin may directly cross-link to the chitosan and have a single unit spacer instead of a polymer spacer which would change the mechanical properties of the hydrogel. Genipin was therefore dissolved in different pH solutions formed of biocompatible buffers. A first buffer was formed from phosphate at a pH between about 7 and about 10 and a second buffer was formed from citrate at a pH between about 4 and about 5. The second buffer was formed from 0.1 M sodium citrate and 0.1 M citric acid.

The next step in fabricating the second population of test sensors was to soak the cured layer of chitosan and glucose oxidase into one or the other of the genipin solutions. One group was soaked for 6 hours in a citrate buffer having a pH of 4.98, and another group was soaked for 6 hours in a phosphate buffer having a pH of 7.54. The exposure created a cross-linking reaction between chitosan and genipin. Then, the matrix was cured again by drying in ambient air over night. The sensors were tested for adhesion, and adhesion after soaking, using the same procedure that was used for the first population.

The results showed excellent adhesion of the hydrogel layer to the electrode surface, even after soaking in a phosphate buffer simulating an intravenous environment. Superior results were obtained from test sensors made from the 4.98 pH genipin buffer. Of these, three out of four test sensors had three or fewer cubes removed as a result of the ASTM adhesion test performed after soaking in the phosphate buffer for at least 24 hours.

The glucose response was tested using another sample from the second population of test samples. This sample was divided into a 4.98-pH group and 7.54-pH group. For simplicity, these are referred to as the 5-pH group and 7-pH group, respectively. After soaking each test sensor from these groups for 15.5 hours in a saline solution, each sensor was exposed to known glucose concentrations, with the working electrode excited to a potential of about 650 mV. For each sensor, the electrical current resulting in the electrode was measured at glucose concentrations of 50, 100, and 150 mg/dL.

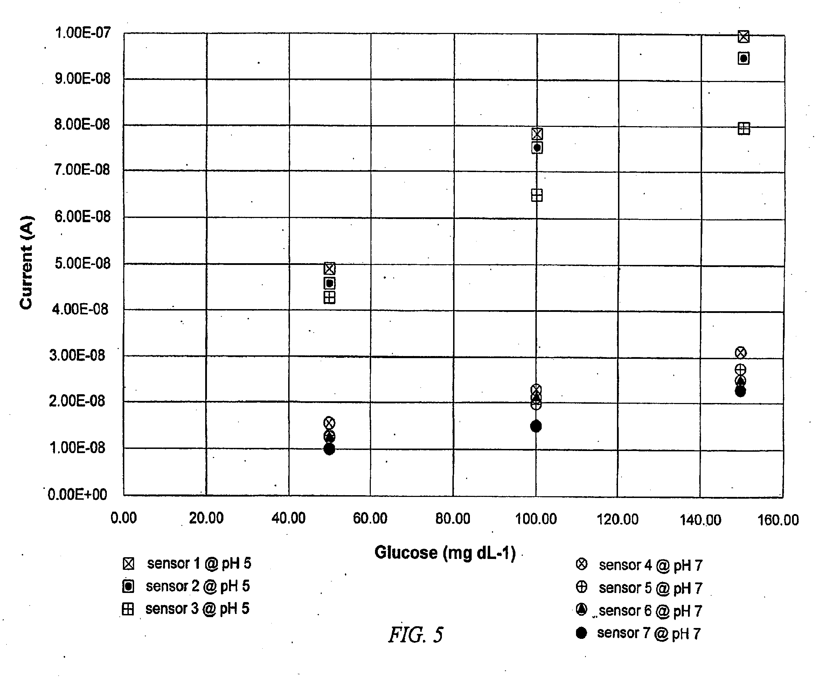

The graph of FIG. 5 shows the results of the glucose assay on seven sensors from the second population. All exhibited linear response characteristics of current as a function of glucose concentration. The 5-pH group exhibited better linearity and superior current output. Because the 5-pH group is believed to have stronger mechanical properties for entrapping glucose oxidase, the results indicate that a higher current response may be obtained by increasing the amount of glucose oxidase that is crosslinked or entrapped within the hydrogel layer.

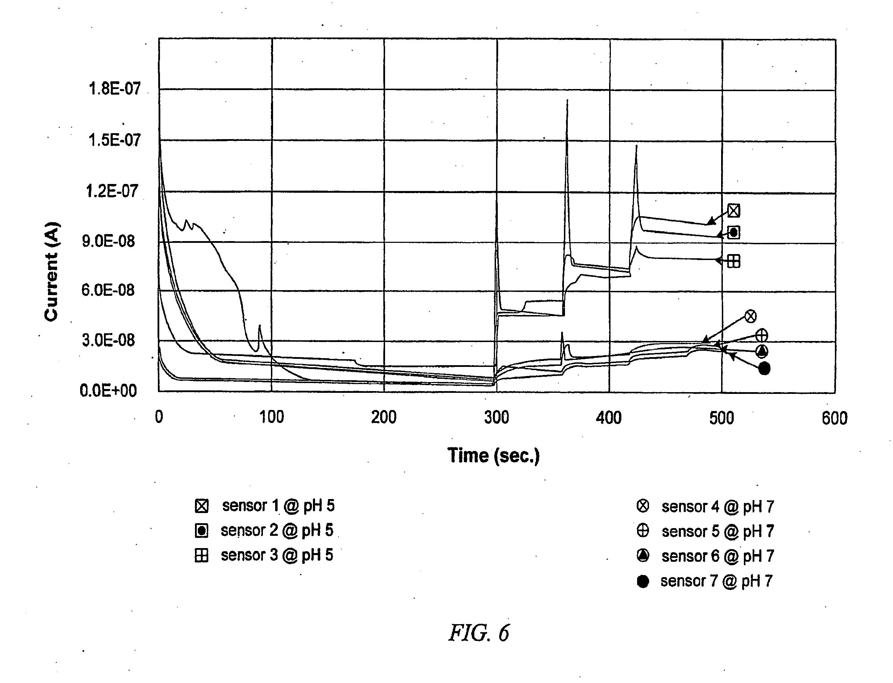

The graph of FIG. 6 shows the results of the glucose assay on the same sensors as a function of current output over time. The time period shown covers the three step changes in glucose concentration that correspond to the 50, 100, and 150 mg/dL concentrations. After an initial transient spike in the current signal coincident with each step change, the response at each concentration quickly levels off to a steady state response. The steady response over time during step changes in glucose levels indicates that the glucose oxidase is more or less evenly dispersed within the hydrogel matrix, and is able to sustain a linear current output over time. Since the adhered layer of glucose oxidase is maintained over time in the presence of a reactant such as glucose, this indicates that the glucose oxidase adhesion is also stable, and is able to maintain contact with glucose and with the electrode while immobilized within the chitosan-genipin matrix.

The linear response exhibited in FIGS. 5 and 6 indicates that a hydrogel according to the invention may also function as a rate-limiting layer 25 between blood plasma and glucose oxidase. That is, the chitosan can be cross-linked in such a manner and be proportion to genipin and glucose oxidase to obtain the desired oxygen sensitivity. The desired oxygen sensitivity is one that allows an abundance of oxygen to pass through the hydrogel layer while selectively passing glucose, to ensure that the oxidation reaction with glucose oxidase is limited by the available glucose.

Finally, there was no significant change in reactivity of a sensor fabricated using anywhere from 1/20,000 to 1/60,000 wt. % glucose oxidase, mixed in a chitosan solution having a concentration in the range of about 0.25 to 1.0 wt. %, provided that the pH of the genipin buffer was constant. Under these conditions, it is believed that a concentration as low as 1/120,000 glucose oxidase in chitosan would provide a measurable and linear current response.

The invention has been disclosed in an illustrative manner. Accordingly, the terminology employed throughout should be read in an exemplary rather than a limiting manner. Although minor modifications of the invention will occur to those well versed in the art, it shall be understood that what is intended to be circumscribed within the scope of the patent warranted hereon are all such embodiments that reasonably fall within the scope of the advancement to the art hereby contributed, and that that scope shall not be restricted, except in light of the appended claims and their equivalents.

## Claims

1. An intravenous amperometric biosensor, comprising:
a flexible substrate;
an electrode bonded to the substrate; and
a hydrogel layer bonded to the electrode, the hydrogel layer including chitosan, at least one oxidase enzyme, preferably glucose oxidase, and genipin cross-linked to the chitosan forming a matrix immobilizing the oxidase enzyme.

2. The biosensor of claim 1, wherein the hydrogel layer provides a rate-limiting layer for selectively diffusing a blood component to react with the oxidase enzyme, preferably glucose oxidase.

3. A method for forming a hydrogel for immobilizing a reactive agent on an electrode, comprising:
dissolving chitosan in an acidic solution;
adding an oxidase to the chitosan solution;
applying the chitosan solution to a surface of the electrode;
curing the applied chitosan solution until it forms a solid film; and
immersing the solid film in a genipin solution to cross-link the chitosan and the genipin, thereby immobilizing the oxidase.

4. The method of claim 3, wherein the dissolving step creates a chitosan concentration of between about 0.1 and about 2.0 weight percent chitosan, preferably a chitosan concentration of about 0.25 weight percent chitosan.

5. The method of claim 3, wherein the oxidase comprises glucose oxidase.

6. The method of claim 3, wherein the adding step creates an oxidase concentration of between about 1/120,000 and about 1/10,000 weight percent oxidase.

7. The method of claim 3, wherein the immersing step comprises immersing the solid film in a genipin solution for a duration of between about 2 and about 10 hours.

8. The method of claim 3, wherein the genipin solution comprises a buffer having a pH between about 4 and about 10.

9. The method of claim 3, wherein the genipin solution comprises about 1 percent weight-to-volume of genipin.

10. The method of claim 3, further comprising adding to the cross-linked genipin and chitosan a second, more densely cross-linked layer of genipin and chitosan to provide a rate-limiting layer for selectively diffusing a blood component to react with the oxidase.

## Patentansprüche

1. Intravenöser amperometrischer Biosensor, umfassend:
ein flexibles Substrat:
eine Elektrode, gebunden an das Substrat, und
eine Hydrogelschicht, gebunden an die Elektrode, wobei die Hydrogelschicht Chitosan, mindestens ein Oxidaseenzym, vorzugsweise Glycoseoxidase, und Genipin, vernetzt an das Chitosan, eine Matrix bildend, welche das Oxidaseenzym immobilisiert, einschließt.

2. Biosensor gemäß Anspruch 1, wobei die Hydrogelschicht eine geschwindigkeitslimitierende Schicht zum selektiven Diffundieren einer Blutkomponente unter Reaktion mit dem Oxidaseenzym, vorzugsweise Glycoseoxidase, bereitstellt.

3. Verfahren zum Bilden eines Hydrogels zum Immobilisieren eines reaktiven Mittels auf einer Elektrode, umfassend:
das Lösen von Chitosan in einer sauren Lösung,
das Zugeben einer Oxidase zu der Chitosanlösung,
das Aufbringen der Chitosanlösung auf eine Oberfläche der Elektrode,
das Härten der aufgebrachten Chitosanlösung, bis sie einen festen Film bildet, und
das Eintauchen des festen Films in eine Genipinlösung, um das Chitosan und
das Genipin zu vernetzen, wodurch die Oxidase immobilisiert wird.

4. Verfahren gemäß Anspruch 3, wobei der Lösungsschritt eine Chitosankonzentration von zwischen etwa 0,1 und etwa 2,0 Gew.-% Chitosan, vorzugsweise eine Chitosankonzentration von etwa 0,25 Gew.-% Chitosan, bewirkt.

5. Verfahren gemäß Anspruch 3, wobei die Oxidase Glucoseoxidase umfaßt.

6. Verfahren gemäß Anspruch 3, wobei der Zugabeschritt eine Oxidasekonzentration von zwischen etwa 1/120000 und etwa 1/10000 Gew.-% Oxidase bewirkt.

7. Verfahren gemäß Anspruch 3, wobei der Eintauchschritt das Eintauchen des festen Films in eine Genipinlösung für eine Zeitdauer von zwischen etwa 2 und etwa 10 Stunden umfaßt.

8. Verfahren gemäß Anspruch 3, wobei die Genipinlösung einen Puffer mit einem pH von zwischen etwa 4 und etwa 10 umfaßt.

9. Verfahren gemäß Anspruch 3, wobei die Genipinlösung etwa 1 Gew.-%, bezogen auf das Volumen, Genipin umfaßt.

10. Verfahren gemäß Anspruch 3, weiter umfassend das Zugeben einer zweiten dichter vernetzten Schicht von Genipin und Chitosan zu dem vernetzten Genipin und Chitosan, um eine geschwindigkeitslimitierende Schicht zum selektiven Diffundieren einer Blutkomponente unter Reaktion mit der Oxidase bereitzustellen.

## Revendications

1. Biocapteur ampérométrique intraveineux, comprenant:
un substrat flexible;
une électrode liée au substrat ; et
une couche d'hydrogel liée à l'électrode, la couche d'hydrogel comprenant du chitosane, au moins une enzyme oxydase, de préférence la glucose oxydase, et de la génipine réticulée au chitosane formant une matrice qui immobilise l'enzyme oxydase.

2. Biocapteur selon la revendication 1, dans lequel la couche d'hydrogel fournit une couche de limitation de la vitesse permettant la diffusion sélective d'un composant sanguin destiné à réagir avec l'enzyme oxydase, de préférence la glucose oxydase.

3. Procédé de formation d'un hydrogel destiné à immobiliser un agent réactif sur une électrode, comprenant:
la dissolution du chitosane dans une solution acide ;
l'ajout d'une oxydase à la solution de chitosane;
l'application de la solution de chitosane sur une surface de l'électrode ;
la polymérisation de la solution de chitosane appliquée jusqu'à ce qu'elle forme un film solide ; et
l'immersion du film solide dans une solution de génipine pour réticuler le chitosane et la génipine afin d'immobiliser l'oxydase.

4. Procédé selon la revendication 3, dans lequel l'étape de dissolution crée une concentration de chitosane comprise entre environ 0,1 et environ 2,0% en poids de chitosane, de préférence une concentration de chitosane d'environ 0,25 % en poids de chitosane.

5. Procédé selon la revendication 3, dans lequel l'oxydase comprend la glucose oxydase.

6. Procédé selon la revendication 3, dans lequel l'étape d'ajout crée une concentration d'oxydase comprise entre environ 1/120 000 et environ 1/10 000 % en poids d'oxydase.

7. Procédé selon la revendication 3, dans lequel l'étape d'immersion consiste à immerger le film solide dans une solution de génipine pendant une durée comprise entre environ 2 heures et environ 10 heures.

8. Procédé selon la revendication 3, dans lequel la solution de génipine comprend un tampon ayant un pH compris entre environ 4 et environ 10.

9. Procédé selon la revendication 3, dans lequel la solution de génipine comprend environ 1 % en poids par volume de génipine.

10. Procédé selon la revendication 3, consistant en outre à ajouter à la génipine et au chitosane réticulés une seconde couche de génipine et de chitosane, plus densément réticulée, pour fournir une couche de limitation de la vitesse permettant la diffusion sélective d'un composant sanguin destiné à réagir avec l'oxydase.
